(19) Europäisches Patentamt
European Patent Office
Office européen des brevets

(11) Veröffentlichungsnummer : **0 611 768 A2**

# (12) EUROPÄISCHE PATENTANMELDUNG

(21) Anmeldenummer : **94810070.6**

(22) Anmeldetag : **08.02.94**

(51) Int. Cl.[5] : **C07D 513/04,** C07C 331/28, C07D 237/04, A01N 43/90, // (C07D513/04, 285:00, 237:00)

(30) Priorität : **17.02.93 CH 486/93**

(43) Veröffentlichungstag der Anmeldung :
**24.08.94 Patentblatt 94/34**

(84) Benannte Vertragsstaaten :
**AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Pissiotas, Georg, Dr.**
**Am Sonnenrain 71**
**D-79539 Lörrach (DE)**
Erfinder : **Moser, Hans, Dr.**
**Hauptstrasse 67 B**
**CH-4312 Magden (CH)**
Erfinder : **Brunner, Hans-Georg, Dr.**
**Wannenstrasse 14**
**CH-4415 Lausen (CH)**

(54) **Phenylimino-thiadiazabicyclononanon-Salze mit herbiziden Eigenschaften.**

(57) Verbindungen der Formel I

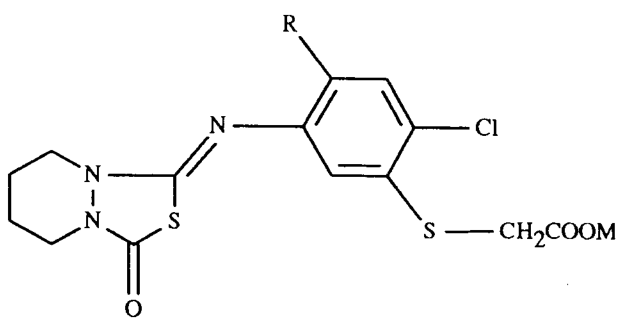

(I),

worin
R Wasserstoff oder Fluor ; und
M ein Alkali- oder Erdalkalimetallion oder ein Ammoniumkation ist, die Herstellung dieser Verbindungen und ihre Verwendung als herbizide Wirkstoffe sind beschrieben.

EP 0 611 768 A2

Die vorliegende Erfindung betrifft neue Phenylimino-thiadiazabicyclononanon-Salze und ihre Verwendung als Herbizid.

Herbizid wirksame Phenylimino-thiadiazabicyclononanon-Derivate sind z.B. aus EP-A-0 238 711, EP-A-0 273 417, EP-A-0 312 064 und JP Patent Kokai Hei 1-186894 bekannt. Diese Derivate weisen unterschiedliche Substituenten am Phenylring auf.

Es wurden nun Salze aus dieser Reihe von Wirkstoffen mit guten herbiziden Eigenschaften gefunden. Gegenstand der vorliegenden Erfindung sind somit die Verbindungen der Formel I

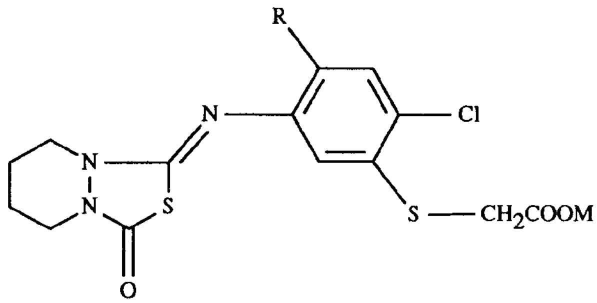

(I),

worin

R Wasserstoff oder Fluor; und

M ein Alkali- oder Erdalkalimetallion oder ein Ammoniumkation ist.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-isopropylamin, Methyl-hexylamin, Methyl-nonylamin, Methyl-pentadecylamin, Methyl-octadecylamin, Ethyl-butylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethylethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Diethanolamin, Trimethylamin, Triethylamin, Tri-n-propylamin, Tri-iso-propylamin, Tri-n-butylamin, Tri-iso-butylamin, Tri-sek.-butylamin, Tri-n-amylamin; heterocyclische Amine wie z.B. Pyridin, Chinolin, iso-Chinolin, Morpholin, Thiomorpholin, N-Methylmorpholin, N-Methyl-thiomorpholin , Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline.

Beispiele für quaternäre Ammoniumbasen sind im allgemeinen die Kationen von Halogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Weitere Gegenstände der vorliegenden Erfindung sind auch herbizide Mittel, welche die erfindungsgemässen Verbindungen als Wirkstoff enthalten, Verfahren zur Herstellung dieser Verbindungen, sowie die Verwendung dieser Verbindungen und Mittel zur selektiven Bekämpfung von Unkräutern.

Das erfindungsgemässe Verfahren zur Herstellung der Verbindungen der Formel I erfolgt in Analogie zu bekannten Verfahren, wie z.B. in EP-A-0 312 064 und EP-A-0 468 924 beschrieben, und ist dadurch gekennzeichnet, dass man eine Verbindung der Formel II

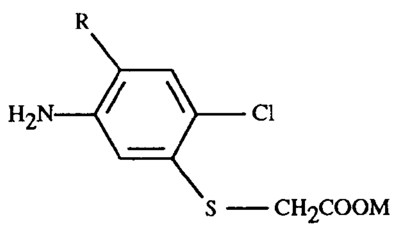

(II)

mit Thiophosgen unter üblichen Bedingungen in das Isothiocyanat der Formel III

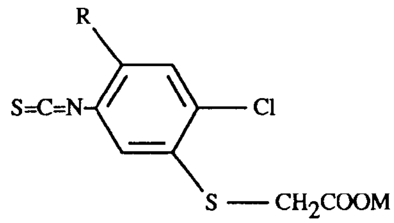

(III)

überführt und dieses anschliessend mit Hexahydropyridazin der Formel V

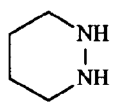

(V)

in die Verbindung der Formel IV

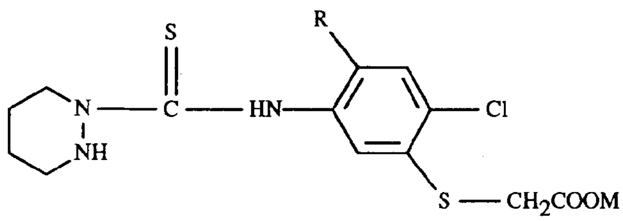

(IV)

umsetzt, wobei in den Verbindungen der Formeln II, III und IV R Wasserstoff oder Fluor; und M ein Alkali- oder Erdalkalimetallion oder ein Ammoniumkation bedeutet, und anschliessend die erhaltene Verbindung der Formel IV mit Phosgen, Diphosgen (Chlorameisensäure-trichlormethylester) oder Triphosgen (Bis-(trichlormethyl)-carbonat) zur Verbindung der Formel I reagieren lässt.

Dieses Herstellungsverfahren wird im folgenden Reaktionsschema beispielhaft näher erläutert.

Reaktionsschema:

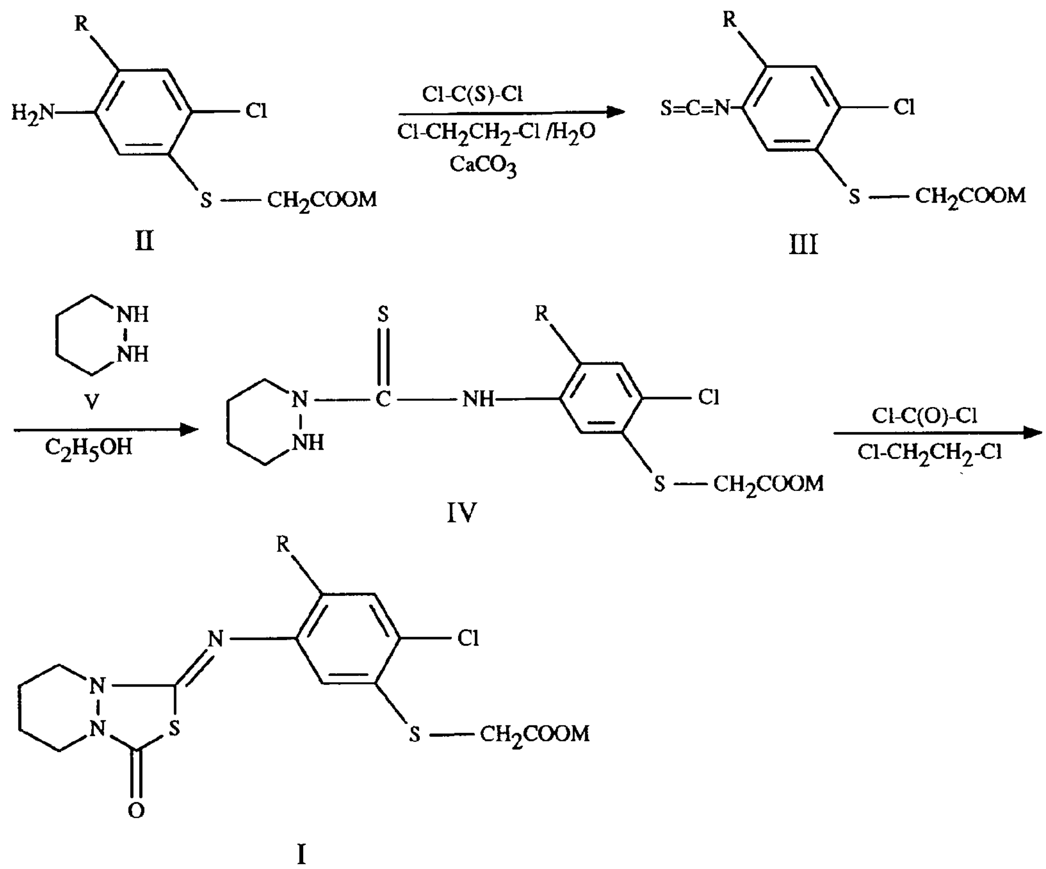


Das Isothiocyanat der Formel III wird in an sich bekannter Weise durch Umsetzen des entsprechenden Amins der Formel II mit Thiophosgen erhalten. Derartige Verfahren sind beispielsweise in EP-A-0 304 920, Seiten 53 und 55; EP-A-0 312 064, Seite 11, letzter Abschnitt und Seite 12, Zeilen 12-18; und EP-A-0 468 924, Seite 21, Zeilen 29-47, Seite 22, Beispiel H2, und Seite 27, Beispiel H17, beschrieben.

Die Verbindung der Formel IV wird in an sich bekannter Weise durch Umsetzen des Isothiocyanats der Formel III mit Hexahydropyridazin der Formel V erhalten. Derartige Umsetzungen sind beispielsweise in EP-A-0 312 064, Seite 11, letzter Abschnitt, Seite 12, Zeilen 19-24; EP-A-0 468 924, Seite 16, letzter Abschnitt und Seite 17, erster Abschnitt, Seite 19, Zeilen 42-46 und Seite 22, Beispiel H3, beschrieben.

Die Cyclisierung des Hexahydropyridazinyl-thiocarbonylamino-Derivats der Formel IV kann zweckmässigerweise mit Phosgen, Diphosgen (Chlorameisensäuretrichlormethylester) oder Triphosgen (Bis-(trichlormethyl)-carbonat) in einem inerten Lösungsmittel bei niedrigen Temperaturen, vorzugsweise 0 bis 50°C, besonders bevorzugt bei 0 bis 15°C, erfolgen. Derartige Cyclisierungen sind beispielsweise in EP-A-0 312 064, Seite 11, Zeilen 1-40 und Herstellungsbeispiele 1 bis 5 auf Seiten 13 und 14; und EP-A-0 468 924, Seite 19, Zeilen 47-51, Seite 23, Beispiel H4 und Seite 28, Beispiel H19, beschrieben.

Die Zwischenprodukte der Formeln III und IV sind neu und wurden speziell für die Synthese der Verbindungen der Formel I entwickelt. Sie bilden daher auch einen Gegenstand der vorliegenden Erfindung.

Die Herstellung der Ausgangsverbindung der Formel II ist in EP-A-0 126 419 beschrieben.

Das Hexahydropyridazin der Formel V ist bekannt oder lässt sich analog zu literaturbekannten Verfahren herstellen, beispielsweise Bull. Soc. Chim. France 1957, 704; EP-A-0 304 920, Seiten 9-11 (Schemata 2-4); und EP-A-0 468 924, Seite 19, letzter Abschnitt, Seite 20, erster Abschnitt und Seite 28, Beispiel H18.

Das Endprodukt der Formel I kann auf übliche Weise durch schonendes Einengen und/oder Verdampfen des Lösungsmittels im Vakuum isoliert und durch Umkristallisieren oder Zerreiben des festen Rückstandes in Lösungsmitteln, in denen es sich nicht gut löst, wie Ether, aromatischen Kohlenwasserstoffe oder chlorierten Kohlenwasserstoffe, gereinigt werden.

Für die erfindungsgemäße Verwendung der Verbindungen der Formel I oder diese enthaltende Mittel kommen alle in der Landwirtschaft üblichen Applikationsmethoden wie z.B. preemergente Applikation, postemergente Applikation und Saatbeizung, sowie verschiedene Methoden und Techniken in Betracht, wie beispielsweise die kontrollierte Wirkstoffabgabe. Dazu wird der Wirkstoff in Lösung auf mineralische Granulatträger oder polymerisierte Granulate (Harnstoff/Formaldehyd) aufgezogen und getrocknet.

Gegebenenfalls kann zusätzlich ein Überzug aufgebracht werden (Umhüllungsgranulate), der es erlaubt, den Wirkstoff über einen bestimmten Zeitraum dosiert abzugeben.

Die Verbindung der Formel I kann in unveränderter Form, d.h. wie sie aus der Synthese anfällt, eingesetzt werden, vorzugsweise verarbeitet man sie aber auf übliche Weise mit den in der Formulierungstechnik üblichen Hilfsmitteln z.B. zu emulgierbaren Konzentraten, direkt versprühbaren oder verdünnbaren Lösungen, verdünnten Emulsionen, Spritzpulvern, löslichen Pulvern, Stäubemitteln, Granulaten oder Mikrokapseln. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen des Wirkstoffs mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen $C_8$ bis $C_{12}$, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside, die auch in den erfindungsgemässen Mitteln verwendet werden können, sind u.a. in folgenden Publikationen beschrieben:

- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache Tensid-Taschenbuch", Carl Hanser Verlag, München/Wien 1981.

Die herbiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

| Emulgierbare Konzentrate: | |
|---|---|
| Aktiver Wirkstoff: | 1 bis 90 %, vorzugsweise 5 bis 50 % |
| oberflächenaktives Mittel: | 5 bis 30 %, vorzugsweise 10 bis 20 % |
| flüssiges Trägermittel: | 15 bis 94 %, vorzugsweise 70 bis 85 % |

| Stäube: | |
|---|---|
| Aktiver Wirkstoff: | 0,1 bis 50 %, vorzugsweise 0,1 bis 1 % |
| festes Trägermittel: | 99,9 bis 90 %, vorzugsweise 99,9 bis 99 % |

| Suspensions-Konzentrate: | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| Benetzbare Pulver: | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

| Granulate: | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Der Wirkstoff der Formel I wird in der Regel mit Aufwandmengen von 0,001 bis 2 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Die Verbindung der Formel I zeichnet sich durch herbizide Eigenschaften aus, die sie ausgezeichnet zum Einsatz in Kulturen von Nutzpflanzen, insbesondere in Getreide, Reis, Mais und Soja befähigen.

Unter Kulturen sind auch solche zu verstehen, die durch konventionelle züchterische oder gentechnologische Methoden gegen Herbizide bzw. Herbizidklassen tolerant gemacht worden sind.

Die nachfolgenden Beispiele erläutern die Erfindung weiter, ohne sie zu beschränken.

1. Herstellung der erfindungsgemässen Verbindungen der Formel I

Beispiel H1: 1-(2-Chlor-4-fluor-5-isothiocyanato-phenylthio)-essigsäure

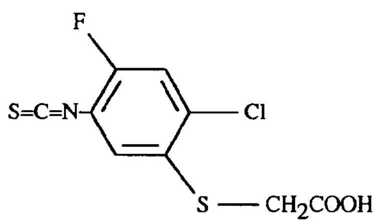

(III)

Zu einem Gemisch aus 7,5 g Calciumcarbonat, 5 ml Thiophosgen in 100 ml Ethylenchlorid und 100 ml Wasser gibt man unter Rühren bei Raumtemperatur eine Lösung von 11,8 g 1-(5-Amino-2-chlor-4-fluorphenylthio)-essigsäure in 100 ml Ethylenchlorid tropfenweise hinzu. Nach sechsstündigem Rühren bei Raumtemperatur werden die festen Anteile der Reaktionsmischung abfiltriert und in einem Essigsäureethylester/Wasser-Gemisch aufgenommen. Unter Rühren versetzt man diese Suspension mit 2N Salzsäurelösung und rührt währen 1/2 Stunde weiter. Die organische Phase wird abgetrennt, über Natriumsulfat getrocknet und anschliessend eingedampft. Man erhält 11,5 g 1-(2-Chlor-4-fluor-5-isocyanato-phenylthio)-essigsäure mit einem Smp. von 118°C.

Beispiel H2: 1-[2-Chlor-4-fluor-5-(1-hexahydropyridazinyl-thiocarbonylamino)-phenylthio]-essigsäure

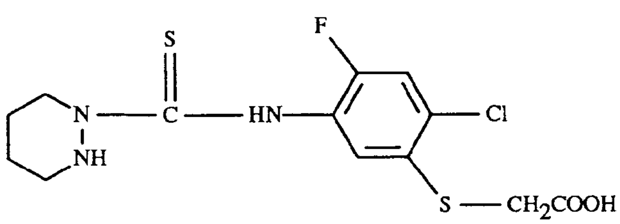

(IV)

Zu einer Lösung aus 5,0 g Hexahydropyridazin in 100 ml Ethanol gibt man unter Rühren bei Raumtemperatur eine Lösung von 11,5 g 1-(2-Chlor-4-fluor-5-isothiocyanatophenylthio)-essigsäure in 100 ml Ethylenchlorid tropfenweise hinzu. Nach dreistündigem Rühren bei Raumtemperatur wird die Reaktionsmischung im Vakuum eingedampft. Man erhält 14,5 g 1-[2-Chlor-4-fluor-5-(1-hexahydropyridazinyl-thiocarbonylamino)-phenylthio]-essigsäure mit einem Smp. von 110-118°C.

Beispiel H3: 9-[4-Chlor-2-fluor-5-(1-oxycarbonylmethylthio)-phenylimino]-8-thia-16-diazabicyclo[4.3.0]nonan-7-on

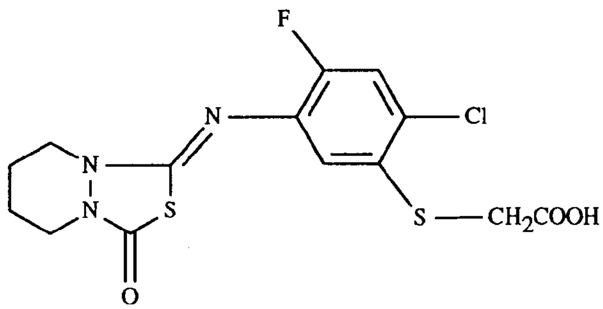

(Ia)

Zu einer 40 ml 20%iger toluolischer Phosgenlösung gibt man unter Rühren bei einer Temperatur von 0-5°C eine Lösung von 14,5 g 1-[2-Chlor-4-fluor-5-(1-hexahydropyndazinyl-thiocarbonylamino)-phenylthio]-essigsäure in 100 ml Ethylenchlorid tropfenweise hinzu. Anschliessend lässt man die Reaktionsmischung bei Raumtemperatur während 6 Stunden rühren und giesst sie dann in Eiswasser. Die organische Phase wird abgetrennt und über Natriumsulfat getrocknet. Nach dem Eindampfen im Vakuum erhält man 15,0 g 9-[4-Chlor-2-fluor-5-(1-oxycarbonylmethylthio)-phenylimino]-8-thia-1,6-diazabicyclo-[4.3.0]nonan-7-on mit einem Smp. von 170-172°C.

Beispiel H4: Natriumsalz von 9-[4-Chlor-2-fluor-5-(1-oxycarbonylmethylthio)-phenylimino]-8-thia-1,6-diazabicyclo[4.3.0]nonan-7-on

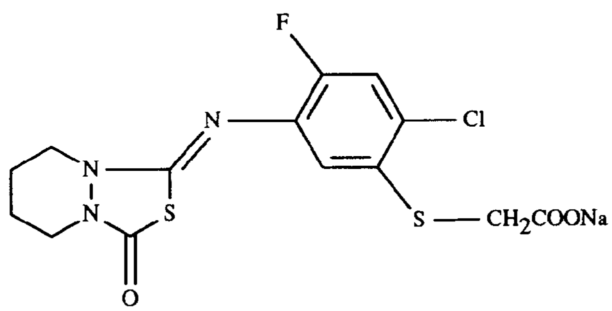

(1.001).

Zu einer Suspension von 1,94 g (0,005 Mol) 9-[4-Chlor-2-fluor-5-(1-oxycarbonylmethylthio)-phenylimino]-8-thia-1,6-diazabicyclo[4.3.0]nonan-7-on in 75 ml Methanol wird 0,28 g Natriummethylat bei Raumtemperatur zugegeben, wobei die Suspension in Lösung geht. Es wird während 15 Minuten bei Raumtemperatur weitergerührt und anschliessend das Lösungsmittel im Vakuum schonend abgedampft. Zurück bleibt ein glasiges Produkt, das in Diethylether digeriert wird. Man erhält 1,7 g (83% Ausbeute) des gewünschten Na-Salzes von 9-[4-Chlor-2-fluor-5-(1-oxycarbonylmethylthio)-phenylimino]-8-thia-1,6-diazabicyclo[4.3.0]nonan-7-on als Pulver; Smp. 203°C.

Beispiel H5: Natriumsalz von 9-[4-Chlor-5-(1-oxycarbonylmethylthio)-phenylimino]-8-thia-1,6-diazabicyclo[4.3.0]nonan-7-on

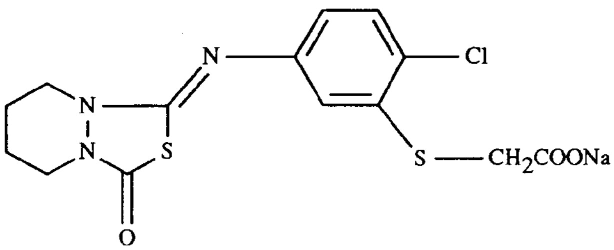

(1.002)

wird analog zu Beispiel H4 erhalten; Smp. 208-215°C.

Auf analoge Weise können auch die in der Tabelle 1 zusammengestellten Verbindungen der Formel I hergestellt werden.

Tabelle 1: Verbindungen der Formel I

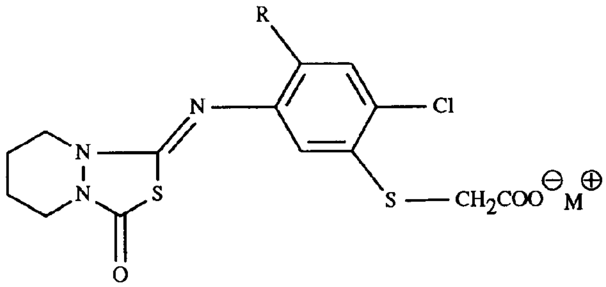

(I)

| Verb.Nr. | R | M | Phys. Daten |
|---|---|---|---|
| 1.001 | F | Na | Smp. 203°C |
| 1.002 | H | Na | Smp. 208-215°C |
| 1.003 | F | 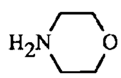 | Smp. 120°C |
| 1.004 | H | 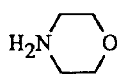 | |
| 1.005 | F | Li | |
| 1.006 | H | Li | |
| 1.007 | F | K | |
| 1.008 | H | K | |
| 1.009 | F | Mg | |
| 1.010 | H | Mg | |
| 1.011 | F | Ca | |
| 1.012 | H | Ca | |
| 1.013 | F | $NH_4$ | |
| 1.014 | H | $NH_4$ | |
| 1.015 | F | $CH_3NH_3$ | |
| 1.016 | H | $CH_3NH_3$ | |
| 1.017 | F | $(CH_3)_2NH_2$ | |
| 1.018 | H | $(CH_3)_2NH_2$ | |
| 1.019 | F | $(CH_3)_3NH$ | |
| 1.020 | H | $(CH_3)_3NH$ | |
| 1.021 | F | $C_2H_5NH_3$ | |
| 1.022 | F | $(C_2H_5)_2NH_2$ | |

| Verb.Nr. | R | M | Phys. Daten |
|---|---|---|---|
| 1.023 | F | $(C_2H_5)_3NH$ | |
| 1.024 | F | $C_4H_9NH_3$ | |
| 1.025 | F | $(C_4H_9)_4N$ | |
| 1.026 | F | $HOCH_2CH_2NH_3$ | |
| 1.027 | H | $HOCH_2CH_2NH_3$ | |
| 1.028 | F | $(HOCH_2CH_2)_2NH_2$ | |
| 1.029 | H | $(HOCH_2CH_2)_2NH_2$ | |
| 1.030 | F | $(HOCH_2CH_2)_3NH$ | |
| 1.031 | H | $(HOCH_2CH_2)_3NH$ | |
| 1.032 | F | $C_6H_5-CH_2NH_3$ | |
| 1.033 | H | $C_6H_5-CH_2NH_3$ | |
| 1.034 | F | $C_6H_5-NH_3$ | |
| 1.035 | H | $C_6H_5-NH_3$ | |
| 1.036 | F | $H_5C_2OCO-CH_2NH_3$ | |
| 1.037 | F | 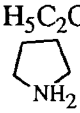 | |
| 1.038 | H | 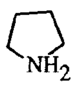 | |
| 1.039 | F | 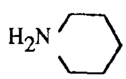 | |
| 1.040 | H | 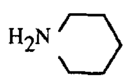 | |
| 1.041 | F | 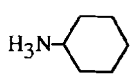 | |
| 1.042 | F | 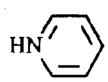 | |
| 1.043 | H | 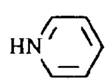 | |
| 1.044 | F | $H_2C=CH-CH_2NH_3$ | |
| 1.045 | H | $H_2C=CH-CH_2NH_3$ | |
| 1.046 | F | $HC\equiv C-CH_2NH_3$ | |
| 1.047 | H | $HC\equiv C-CH_2NH_3$ | |

2. Formulierungsbeispiele für die Verbindung der Formel I (% = Gewichtsprozent)

| F1. Spritzpulver | a) | b) | c) |
|---|---|---|---|
| Verbindung der Formel I | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F2. Emulsions-Konzentrat | |
|---|---|
| Verbindung der Formel I | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F3. Stäubemittel | a) | b) |
|---|---|---|
| Verbindung der Formel I | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| F4. Extruder-Granulat | |
|---|---|
| Verbindung der Formel I | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschließend im Luftstrom getrocknet.

| F5. Umhüllungs-Granulat | |
|---|---|
| Verbindung der Formel I | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

| F6. Suspensions-Konzentrat | |
|---|---|
| Verbindung der Formel I | 40 % |
| Propylenglykol | 10 % |
| Nonylphenolpolyethylenglykolether (15 Mol AeO) | 6 % |
| Na-Ligninsulfonat | 10 % |
| Carboxymethylcellulose | 1 % |
| Silikonöl in Form einer 75%-igen wäßrigen Emulsion | 1 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## 3. Biologische Beispiele

### Beispiel B1: Herbizidwirkung vor dem Auflaufen der Pflanzen (pre-emergent)

Dikotyle Testpflanzen werden in Kunststofftöpfen in Standarderde angesät. Unmittelbar nach der Saat werden die Prüfsubstanzen in wäßriger Suspension, hergestellt aus einem 25 %igen Spritzpulver (Beispiel F1, a)), entsprechend der Dosierung von 2 kg AS/ha aufgesprüht (500 l Wasser/ha). Anschließend werden die Testpflanzen im Gewächshaus unter optimalen Bedingungen kultiviert. Nach 3 Wochen Testdauer wird der Versuch ausgewertet mit einer neunstufigen Notenskala (1 = vollständige Schädigung, 9 = keine Wirkung). Boniturnoten von 1 bis 4 (insbesondere 1 bis 3) bedeuten eine gute bis sehr gute Herbizidwirkung.

Tabelle B1: Pre-emergente Wirkung:

| Wirkstoff der Formel | Testpflanze: | | | | |
|---|---|---|---|---|---|
| | Sinapis | Solanum | Ipomoea | Setaria | Cyperus |
| 1.001 | 1 | 1 | 4 | 2 | 3 |
| 1.002 | 1 | 1 | 4 | 2 | 3 |
| 1.003 | 1 | 1 | 1 | 4 | 2 |

Dieselben Resultate werden erhalten, wenn man die Verbindungen der Formel I gemäß den Beispielen F2 bis F6 formuliert.

### Beispiel B2: Post-emergente Herbizid-Wirkung (Kontaktherbizid)

Dikotyle Testpflanzen werden im Gewächshaus in Kunststofftöpfen mit Standarderde angezogen und im 4- bis 6-Blattstadium mit einer wässrigen Suspension der Prüfsubstanzen, hergestellt aus einem 25 %igen Spritzpulver (Beispiel F1, a)), besprüht, entsprechend einer Dosierung von 2 kg AS/ha (500 l Wasser/ha). Anschliessend werden die Testpflanzen im Gewächshaus unter optimalen Bedingungen weiterkultiviert. Nach ca. 18 Tagen Testdauer wird der Versuch ausgewertet mit einer neunstufigen Notenskala (1 = vollständige Schädigung, 9 = keine Wirkung). Boniturnoten von 1 bis 4 (insbesondere 1 bis 3) bedeuten eine gute bis sehr gute Herbizidwirkung.

Tabelle B2: Post-emergente Wirkung

| Wirkstoff der Formel | Testpflanze: | | | | | |
|---|---|---|---|---|---|---|
| | Sinapis | Solanum | Ipomoea | Setaria | Stellaria | Cyperus |
| 1.001 | 1 | 1 | 1 | 3 | 2 | 3 |
| 1.002 | 1 | 1 | 1 | 3 | 2 | 3 |
| 1.003 | 1 | 1 | 1 | 1 | 3 | 4 |

Dieselben Resultate werden erhalten, wenn man die Verbindungen der Formel I gemäß den Beispielen F2 bis F6 formuliert.

## Patentansprüche

1. Verbindungen der Formel I

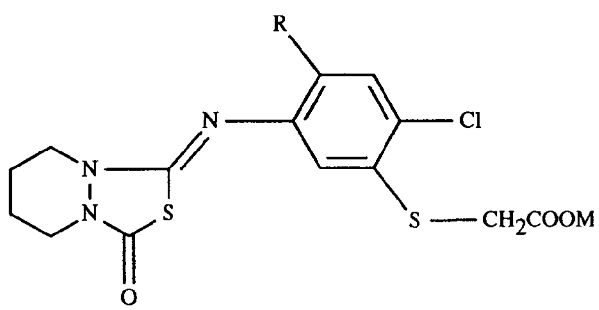

(I),

worin
R Wasserstoff oder Fluor; und
M ein Alkali- oder Erdalkalimetallion oder ein Ammoniumkation ist.

2. Verfahren zur Herstellung der Verbindungen der Formel I gemäss Anspruch 1, dadurch gekennzeichnet, dass man eine Verbindung der Formel II

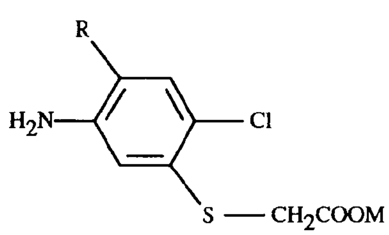

(II)

mit Thiophosgen unter üblichen Bedingungen in die Verbindung der Formel III

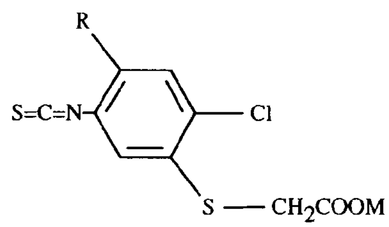

(III)

überführt und diese anschliessend mit dem Hexahydropyridazin der Formel V

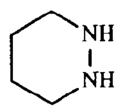

(V)

in die Verbindung der Formel IV

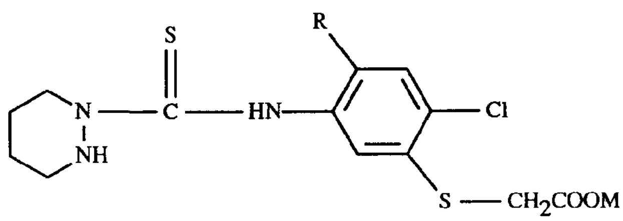

(IV)

umsetzt, wobei in den Verbindungen der Formeln II, III und IV R Wasserstoff oder Fluor; und M ein Alkali- oder Erdalkalimetallion oder ein Ammoniumkation bedeutet, und diese anschliessend mit Phosgen, Diphosgen oder Triphosgen zur Verbindung der Formel I reagieren lässt.

**3.** Verbindungen der Formel III

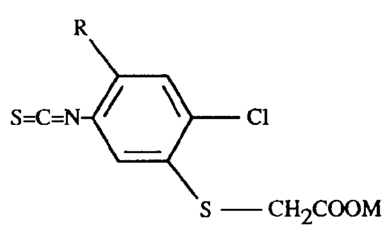

(III),

worin R Wasserstoff oder Fluor; und M ein Alkali- oder Erdalkalimetallion oder ein Ammoniumkation ist.

**4.** Verbindungen der Formel IV

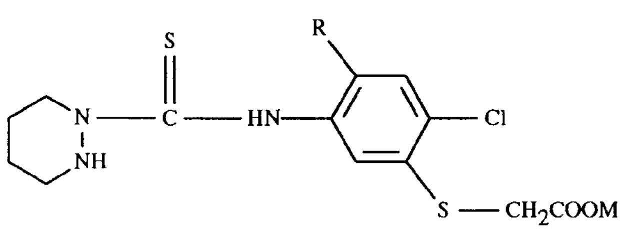

(IV),

worin R Wasserstoff oder Fluor; und M ein Alkali- oder Erdalkalimetallion oder ein Ammoniumkation ist.

**5.** Herbizides Mittel, dadurch gekennzeichnet, dass es die Verbindungen der Formel I gemäss Anspruch 1

enthält.

6. Mittel gemäss Anspruch 5, dadurch gekennzeichnet, dass es 0,1 bis 95% der Verbindungen der Formel I, 1 bis 99,9% eines festen oder flüssigen Zusatzstoffes und 0 bis 25%, insbesondere 0,1 bis 25% eines Tensides enthält.

7. Verfahren zur Bekämpfung unerwünschten Pflanzenwachstums, dadurch gekennzeichnet, dass man die Verbindungen der Formel I gemäss Anspruch 1 oder ein diese Verbindungen enthaltendes Mittel in einer wirksamen Menge auf die Pflanzen oder deren Lebensraum appliziert.

8. Verfahren gemäss Anspruch 7, dadurch gekennzeichnet, dass man die Verbindungen der Formel I in einer Menge von 0,001 bis 2 kg pro Hektar appliziert.

9. Verfahren gemäss Anspruch 7 zur selektiven pre- oder post-emergenten Bekämpfung von Unkräutern in Nutzpflanzenkulturen, insbesondere Getreide, Reis, Mais und Soja.

10. Verwendung der Verbindungen der Formel I gemäss Anspruch 1 zur selektiven pre- oder post-emergenten Bekämpfung von Unkräutern in Nutzpflanzenkulturen, insbesondere Getreide, Reis, Mais und Soja.

11. Verwendung eines Mittels gemäss Anspruch 5 zur selektiven pre- oder post-emergenten Bekämpfung von Unkräutern in Nutzpflanzenkulturen, insbesondere Getreide, Reis, Mais und Soja.